# EUROPEAN PATENT APPLICATION

(11) **EP 1 961 730 A1**
(43) Date of publication of application: **27.08.2008**
(21) Application number: 06834619.6
(22) Date of filing: 13.12.2006
(51) Int. Cl.: C07C 67/08, C07C 67/52, C07C 69/54

(54) **METHOD FOR PRODUCING POLYMERIZABLE HYDROXYDIAMANTYL ESTER COMPOUND**

(30) Priority: 16.12.2005 JP 2005363896
(71) Applicant: Tokuyama Corporation, Shunan-shi, Yamaguchi 745-8648 (JP)
(72) Inventor: MAEHARA, Takayuki, Shunan-shi, Yamaguchi 7458648 (JP)
(74) Representative: Ziebig, Marlene
(86) International application number: PCT/JP2006/324864
(87) International publication number: WO 2007/069656

(57) **Abstract**

The present invention discloses a method for producing a polymerizable hydroxydiamantyl ester compound, which comprises di-halogenating a raw material compound having a diamantane skeleton, such as diamantane or the like, then hydrolyzing the di-halogenation product to produce a 4,9-diamantanediol compound, thereafter esterifying the 4,9-diamantanediol compound in a mixture of a polymerizable unsaturated carboxylic acid and a polymerizable unsaturated carboxylic acid anhydride in the presence of a polymerization inhibitor and an acid catalyst, to obtain a polymerizable hydroxydiamantyl ester compound.

## Description

### Technical Field

The present invention relates to a method for producing a polymerizable hydroxydiamantyl ester compound which is useful as a raw material monomer for functional material or electronic material.

### Background Art

Diamantane derivatives have a skeleton similar to that of adamantane derivatives and have characteristics of superior heat resistance and high transparency. The diamantane skeleton, as compared with the adamantane skeleton, has a large number of rings in the condensed ring. For this reason, the diamantane derivatives are considered to be superior to the adamantane derivatives in physical properties such as heat resistance and the like; consequently, it is expected to apply the diamantane derivatives to highly functional materials (e.g. heat-resistance polymer) or electronic materials (e.g. semiconductor resist). Of the diamantane derivatives, a polymerizable hydroxydiamantyl ester compound is expected as a very useful compound. This compound is used by itself or together with other monomer, as a raw material monomer for production of polymer material or resist material.

In general, the number of compounds having a diamantane skeleton (diamantane compounds), available industrially or as a reagent is limited. In order to synthesize a polymerizable hydroxydiamantyl ester compound, it is necessary to use, as the starting material, any of such a small number of diamantane compounds. However, owing to the difficulty of procurement of diamantane compound, there are many unknown points regarding the reactivity associated with the esterification of the diamantane compound.

As the method for producing a polymerizable hydroxydiamantyl ester compound, there are disclosed the following two methods in, for example, patent literature 1. In the first method, diamantanediol and methacrylic chloride are reacted with each other in the presence of triethylamine. In the second method, diamantanediol and methacrylic acid are reacted with each other in the presence of dicyclohexylcarbodiimide and 4-dimethylaminopyridine.

In any of these methods described in the literature, however, the position of the substituent group bonding to diamantane skeleton is not clarified; therefore, the chemical structure of the polymerizable hydroxydiamantyl ester compound obtained is unclear. Further, in the methods of the patent literature 1, it is described that the reaction requires a long time of 28 hours or 50 hours or more and, after the reaction, purification by column chromatography is required.

We tried actual production of a polymerizable hydroxydiamantyl ester compound from a 4,9-diamantanediol compound (a raw material) under the reaction conditions of 40°C and 7 hours, based on the methods described in the patent literature 1. However, substantially no reaction occurred and no intended product could be obtained.

Meanwhile, when a polymerizable hydroxyadamantyl ester compound is produced from 1,3-adamantanediol (a raw material) which is similar in the structure, a mono-ester can be obtained at a high yield at a high selectivity (see, for example, patent literature 2).

The reason for the above difference is presumed as follows. That is, the 4,9-diamantanediol compound, as compared with the 1,3-adamantanediol compound, is inferior in solubility in organic solvent. Therefore, it is presumed that the reaction rate of the 4,9-diamantanediol compound and the esterifying agent used is extremely small and substantially no intended product could be obtained.

Meanwhile, in general, as the reaction time is made longer, the reaction tends to be promoted. However, as shown in the reason given later, in the esterification of the 1,3-adamantanediol compound, it is known that the rate of the second-stage di-esterification, as compared with the rate of the first-stage mono-esterification, is strikingly low (see patent literature 2, paragraph 35); meanwhile, in the case of the 4,9-diamantanediol compound, it is presumed that the second-stage esterification proceeds similarly to the first-stage esterification, making low the selectivity of intended product.

When a polymerizable hydroxyadamantyl ester compound is produced from 1,3-adamantanediol (a raw material compound), the positions of the two hydroxyl groups in the molecule of raw material compound are relatively near from each other and, therefore, it is considered that there occur, in the introduction of second ester group after the introduction of first ester group, slight steric hindrance and the reduction in nucelophilicity of residual hydroxyl group, caused by the introduction of first ester group. For these steric and electronic reasons, the rate of the esterification of second hydroxyl group after the esterification of first hydroxyl group is low as compared with the rate of the esterification of first hydroxyl group. As a result, it is considered that an intended mono-esterification product can be obtained at a high selectivity.

In contrast, when a polymerizable hydroxydiamantyl ester compound is produced from 4,9-diamantanediol (a raw material), the two hydroxyl groups in the molecule are far apart. Consequently, the steric hindrance when the second ester group is introduced, is small. Further, the electronic influence due to the introduction of first ester group is small as well. As described above, diamantane derivatives and adamantane derivatives differ in steric and electronic environments. Therefore, when the reaction is conducted for a long time, it is difficult to increase the selectivity in the reaction step. As a result, the amount of di-ester compound formed increases, making necessary the purification by column chromatography as described in the patent literature 1. Further, the long-time reaction is not preferred because it may cause various side reactions besides the di-esterification, making complicated the purification.

Similar results are anticipated also when a good solvent for diamantanediol compound is used or a higher reaction temperature is used.

As described above, diamantane derivatives and adamantane derivatives are similar in their carbon skeletons but are largely different in the reactivity, particularly in the reactivity of second-stage esterification; and it is difficult at the present stage to anticipate the reactivity of diamantane derivative.
Patent literature 1: WO 2005/036265
Patent literature 2: JP-A-2001-192355

### Disclosure of the Invention

As described above, in the conventional methods for producing a polymerizable hydroxydiamantyl ester compound, there are a problem of requiring a long time in the reaction and a problem of requiring purification by column chromatography which is relatively small in treating amount. Therefore, it is desired to develop a production method which is free from these problems and can be applied industrially.

The present invention aims at providing a method for producing a polymerizable hydroxydiamantyl ester compound efficiently.

The present inventor made a study in order to solve the above problems. As a result, the present inventor thought, for mono-esterification of 4,9-diamantanediol compound, of a method of esterifying a 4,9-diamantanediol compound in a mixture of a polymerizable unsaturated carboxylic acid and a polymerizable unsaturated carboxylic acid anhydride in the presence of a polymerization inhibitor and an acid catalyst. This method makes possible the mono-esterification of 4,9-diamantanediol compound at a high selectivity and at a high yield. Further, as a method for obtaining the 4,9-diamantanediol compound (a raw material used in the above reaction), there are employed di-halogenation of a diamantane compound (a starting material) and subsequent hydrolysis reaction of di-halogenation product.

The present inventor found that these methods allows for efficient production of a polymerizable hydroxydiamantyl ester compound from a raw material of relatively good availability. The finding has led to the completion of the present invention.

That is, the present invention is a method for producing a polymerizable hydroxydiamantyl ester compound represented by the following formula (1) 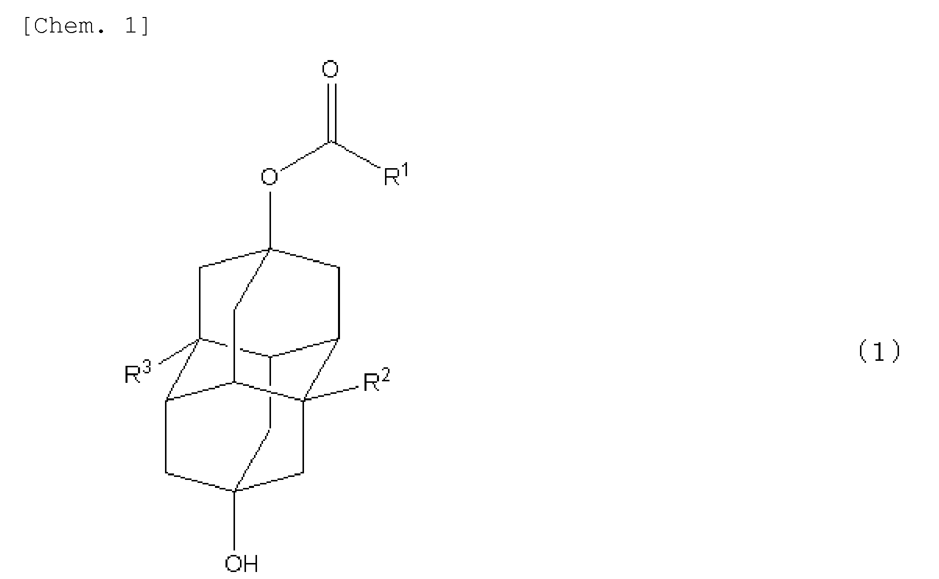 (wherein R¹ is a polymerizable unsaturated hydrocarbon group which may have a substituent, and R² and R³ are each independently a hydrogen atom or an alkyl group of 1 to 5 carbon atoms), which comprises:
(i) a step of di-halogenating a diamantane compound represented by the following formula (2) 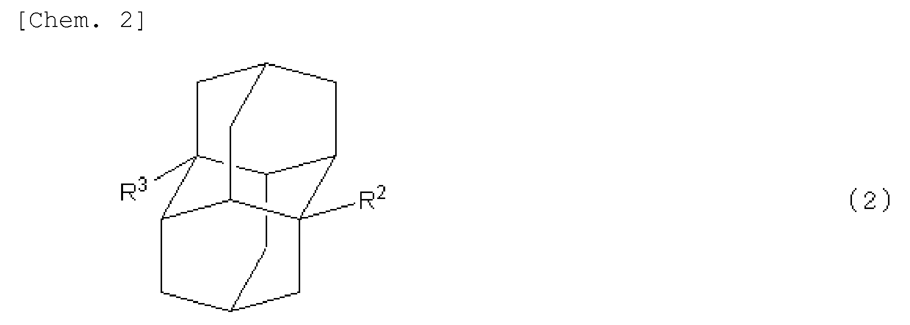 {wherein R² and R³ have each the same definition as for the R² and R³ in the above formula (1)} to obtain a 4,9-dihalogenated diamantane compound,
(ii) a step of hydrolyzing the 4,9-di-halgenated diamantane compound obtained in the above step, to obtain a 4,9-diamantanediol compound, and
(iii) a step of esterifying the 4,9-diamantanediol compound obtained in the above step, in a mixture of a polymerizable unsaturated carboxylic acid represented by the following formula (3) and a polymerizable unsaturated carboxylic acid anhydride represented by the following formula (4) 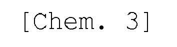 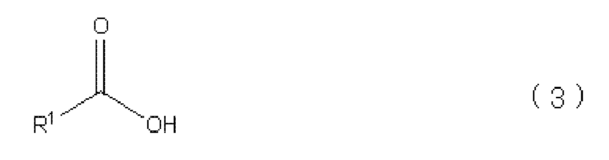 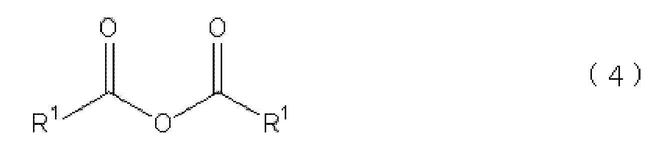 (wherein R¹S are each a polymerizable unsaturated hydrocarbon group which may have a substituent, and they may be the same or different from each other) in the presence of a polymerization inhibitor and an acid catalyst, to obtain a crude product of polymerizable hydroxydiamantyl ester compound represented by the above formula (1).

In the present production method, the steps (i) and (ii) are employed for production of 4,9-diamantanediol compound; therefore, the 4,9-diamantanediol compound can be obtained at a high selectivity. As a result, a polymerizable hydroxydiamantyl ester compound can be produced efficiently from a diamantane compound (a starting material) .

Incidentally, when there is employed a method of directly oxidizing a diamantane compound to obtain diamantanediol, a mixture of diamantanediol and diamantanetriol is obtained, as described in, for example, WO 2005/036265. In this case, a purification step by column chromatography is needed additionally in order to increase the purity of diamantanediol.

Further, the method of step (iii) is employed for esterification; as a result, despite the strikingly low solubility of 4,9-diamantanediol compound in organic solvent, the reaction is over in a short time of within 24 hours.

In the production method of the present invention, even when the obtained crude product containing a polymerizable hydroxydiamantyl ester compound contains a relatively large amount of 4,9-di-ester by-products such as 4,9-bis(methacryloyloxy)diamantane and the like, the polymerizable hydroxydiamantyl ester compound can be obtained efficiently at a high purity by crystallization using a solvent containing an aromatic hydrocarbon. In this case, no complicated separation step such as column chromatography or the like is required.

### Brief Description of the Drawings

Fig. 1 is the proton NMR (¹H-NMR) spectrum of the 9-hydroxy-4-diamantyl methacrylate produced in Example 1.
Fig. 2 is the ¹³C NMR (¹³C-NMR) spectrum of the 9-hydroxy-4-diamantyl methacrylate produced in Example 1.

### Best Mode for Carrying Out the Invention

### Step (i)

In the production method of the present invention, first, a diamantane compound represented by the following formula (2) is di-halogenated to obtain a 4,9-dihalogenated diamantane compound.

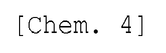

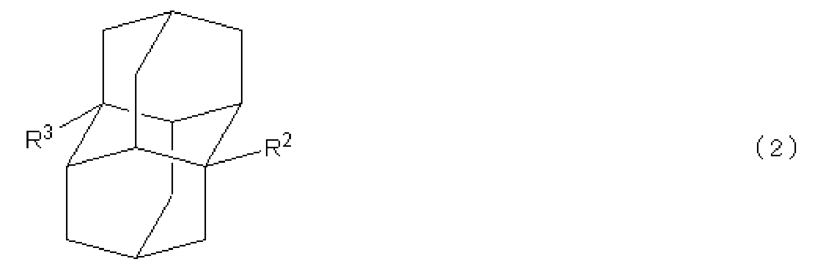

In the formula (2), R² and R³ are each independently a hydrogen atom or an alkyl group of 1 to 5 carbon atoms.

As specific examples of the alkyl group of 1 to 5 carbon atoms, there can be mentioned methyl group, ethyl group, n-propyl group, isopropyl group, n-butyl group, sec-butyl group, tert-butyl group, n-pentyl group, sec-pentyl group, isopentyl group, etc.

In the present step (i), R² and R³ may be appropriately selected so as to correspond to the polymerizable hydroxydiamantyl ester compound to be produced. As specific examples of the diamantane compound usable, there can be mentioned diamantane, 1-methyldiamantane, 1-ethyldiamantane, 1,6-dimethyldiamantane, 1,6-diethyldiamantane, etc. Of these, diamantane is particularly preferred from the standpoints of the availability, the usefulness of the polymerizable hydroxydiamantyl ester compound obtained finally, etc.

In the step (i), a known halogenating agent may be used in order to di-halogenate the diamantane compound. As the halogenation, chlorination is most preferred from the standpoint of the availability of halogenating agent used, the easiness of disposal of waste solution, etc. As the chlorinating agent, a chlorinating agent ordinarily used in chlorination of organic compound can be used with no restriction. As the halogenating agent, a halosulfonic acid (XSO₃H, X is halogen atom) is also preferred in view of the yield of reaction. Chlorosulfonic acid is preferred because a 4,9-dichlorodiamantane compound can be obtained at the highest yield. As other halogenating agents, there can be mentioned bromosulfonic acid, iodosulfonic acid, etc. A case using chlorosulfonic acid is explained below. A case using other halosulfonic acid can also be explained substantially in the same manner.

When chlorosulfonic acid is used as the chlorinating agent, the chlorosulfonic acid can function also as a reaction solvent. When chlorosulfonic acid is used as the chlorinating agent, the use amount of chlorosulfonic acid is preferably 2 to 50 moles, more preferably 3 to 25 moles relative to 1 mole of diamantane compound, in view of the balance of advantage of functioning as a reaction solvent as well as promoting the rate of reaction and the easiness of post-reaction treatment.

The 4,9-dichlorodiamantane compound is slightly soluble in chlorosulfonic acid. In production of 4,9-dichlorodiamantane compound, there is a case that the 4,9-dichlorodiamantane compound slightly dissolved is chlorinated further and a 1,4,9-trichlorodiamantane compound is formed as a by-product. In order to suppress this trichlorination reaction, it is preferred to allow concentrated sulfuric acid (a poor solvent for 4,9-dichlorodiamantane compound) to be present in the reaction system.

Owing to the presence of concentrated sulfuric acid, the 4,9-dichlorodiamantane compound formed by the reaction of diamantane compound with chlorosulfonic acid is separated out in the reaction mixture and eliminated outside from the reaction system; as a result, trichlorination is unlikely to occur. Consequently, the 4,9-dichlorodiamantane compound can be obtained at a high selectivity. There is no particular restriction as to the mixing method of concentrated sulfuric acid, but addition of chlorosulfonic acid to a suspension of diamantane compound in concentrated sulfuric acid is preferred ordinarily.

Here, there is no particular restriction as to the use amount of concentrated sulfuric acid. However, in view of the large yield of 4,9-dichlorodiamantane and the easiness of post-treatment, the use amount is preferably 0.1 to 20 parts by mass, more preferably 0.5 to 10 parts by mass relative to 1 part by mass of diamantane compound.

In the step (i), for example, a diamantane compound and chlorosulfonic acid are reacted to obtain a 4,9-dichlorodiamantane compound. When concentrated sulfuric acid is allowed to be present in the dichlorination system, the water contained in concentrated sulfuric acid may react with chlorosulfonic acid, generating a heat and causing bumping. In order to prevent such accident, it is preferred to add chlorosulfonic acid to the reaction system in divided portions. In this case, the addition may be made in desired times as long as it is two times or more. As to the timing of the second or later addition of chlorosulfonic acid, there is no particular restriction; however, a new fraction of chlorosulfonic acid may be added at a timing at which substantial stoppage of reaction has been confirmed by monitoring the progress of reaction by, for example, gas chromatography (hereinafter referred to simply as GC). There is no particular restriction, either, as to the amounts to be divided; however, when, for example, 5 moles of chlorosulfonic acid are added relative to 1 mole of a diamantane compound, it is possible that 2 moles of chlorosulfonic acid are add at the start of reaction and the remaining 3 moles are added at a timing when substantially no progress of reaction has been confirmed by GC monitoring.

In the step (i), when a diamantane compound and chlorosulfonic acid are reacted with each other with concentrated sulfuric acid being allowed to be present as necessary, to obtain a 4,9-dichlorodiamantane compound, it is preferred to allow an inorganic salt to be present in the reaction system. The presence of an inorganic salt in the reaction system increases the purity of the 4,9-dichlorodiamantane compound obtained and reduces the coloring, etc. of the product. As a specific example of the inorganic salt, a known inorganic salt can be used with no restriction. As the inorganic salt, there can be mentioned, for the availability, metal chlorides such as sodium chloride, potassium chloride, magnesium chloride, calcium chloride and the like; metal carbonates such as lithium carbonate, sodium carbonate, magnesium carbonate, potassium carbonate, calcium carbonate and the like; sulfates such as lithium sulfate, sodium sulfate, magnesium sulfate, potassium sulfate, calcium sulfate and the like; and so forth. Of these inorganic salts, sulfates are preferred and sodium sulfate is particularly preferred for the high purity of the 4,9-dichlorodiamantane compound obtained and the high decreasing effect of coloring.

As to the use amount of the inorganic salt, there is no particular restriction. Too large an amount results in a low reaction rate, and too small an amount results in no effect of inorganic salt addition. The inorganic salt is added ordinarily in an amount of preferably 0.01 to 50 moles, particularly preferably 0.05 to 10 moles relative to 1 mole of the diamantane compound used. As to the addition timing of the inorganic salt, there is no particular restriction, either. However, addition to the reaction system at the start of reaction is ordinarily preferred for simple operation.

In the step (i), there is no particular restriction as to the temperature of the reaction between diamantane compound and chlorosulfonic acid, and the reaction temperature may be appropriately set depending upon the 4,9-dichlorodiamantane compound obtained. Ordinarily, as the reaction temperature is lower, the reaction rate is lower and the solubilities of diamantane compound and formed 4,9-dichlorodiamantane compound in reaction mixture are lower. As a result, the number of chlorine atoms introduced into dichlorodiamantane compound is smaller. When the reaction temperature is high, an opposite state appears and the number of chlorine atoms introduced is larger. In the present invention, the reaction temperature is preferably 0 to 60°C, more preferably 5 to 50°C from the standpoint of the level of the selectivity of the 4,9-dichlorodiamantane compound formed.

There is no particular restriction as to the reaction time of diamantane compound and chlorosulfonic acid. However, a sufficient conversion can be obtained by a reaction of 4 to 48 hours, ordinarily 24 hours.

The equipment used in the above reaction, preferably has a structure in which the equipment inside is in no contact with the atmosphere of outside the equipment, in order to prevent the reaction of chlorosulfonic acid with water and resultant decomposition accompanying generation of acidic gas. It is further preferred that the equipment inside is beforehand purged sufficiently with an inert gas (e.g. nitrogen) and dried and the reaction is conducted while an inert gas (e.g. nitrogen) is being blown into the equipment.

Also, in the step (i), an organic solvent may be added besides the chlorosulfonic acid, the concentrated sulfuric acid and the inorganic salt, to conduct the reaction. As the organic solvent, a halogenated aliphatic hydrocarbon such as methylene chloride, chloroform, carbon tetrachloride or the like is preferred for the low reactivity with chlorosulfonic acid, concentrated sulfuric acid, etc.

The thus-obtained 4,9-dihalogenated diamantane compound is a 4,9-dihalogenated diamantane compound represented by the following formula (5) 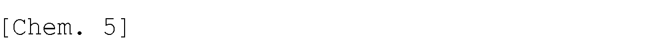 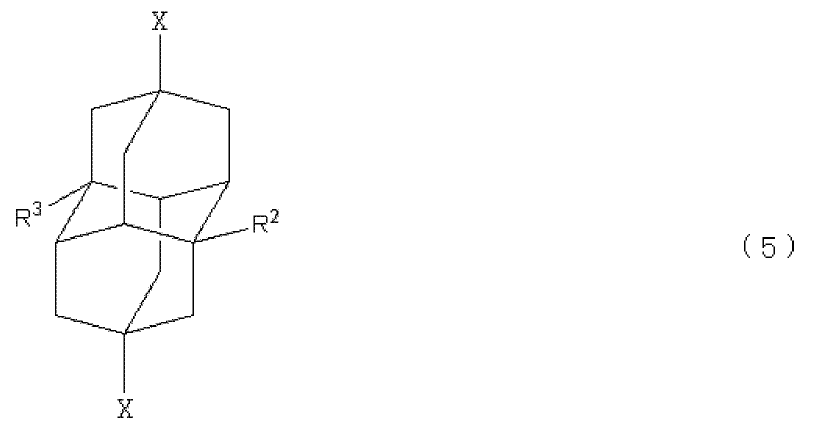 {wherein R² and R³ have the same definitions as for the R² and R³ in the formula (1), and X is a halogen atom}.

The chemical structure of the 4,9-dihalogenated diamantane compound represented by the formula (5) corresponds to the chemical structure of the raw material compound represented by the formula (2) and is different only in the X portions. As specific examples of the formula (5) compound, there can be mentioned 4,9-dichlorodiamantane, 4,9-dibromodiamantane, 4,9-diiododiamantane, 1-methyl-4,9-dichlorodiamantane, 1,6-dimethyl-4,9-dichlorodiamantane, 1-ethyl-4,9-dichlorodiamantane, 1,6-diethyl-4,9-dichlorodiamantane, etc.

There is no particular restriction as to the method for isolating the 4,9-dihalogenated diamantane compound formed after the reaction step (i). However, the following method can be employed when a halosulfonic acid, for example is used as the halogenating agent.

That is, the reaction mixture after the reaction is cooled to room temperature or lower. Then, water is added thereto while the temperature of the system is maintained at 30°C or lower, to decompose the remaining halosulfonic acid. Thereafter, an organic solvent such as dichloromethane, chloroform or the like, capable of dissolving the 4,9-dihalogenated diamantane compound is added to the reaction mixture to extract the 4,9-dihalogenated diamantane compound. The resulting organic solvent containing the 4,9-dihalogenated diamantane compound is washed with an aqueous basic solution or the like, and the neutrality of the organic solvent is confirmed. The organic solvent is concentrated to obtain a crude product of 4,9-dihalogenated diamantane compound (the crude product ordinarily contains a 4,9-dihalogenated diamantane compound (an intended product) in an amount of 70% or more in terms of GC purity.

When the 4,9-dihalogenated diamantane compound is hardly dissolved in an organic solvent and the extraction thereof with the organic solvent is difficult, it is possible to use a means such as filtration, centrifugation or the like to isolate the 4,9-dihalogenated diamantane compound.

The 4,9-dihalogenated diamantane compound obtained as above contains a small amount of impurities and can be used per se sufficiently in the next step. However, it is possible to apply an adsorption treatment such as active carbon treatment, silica treatment, alumina treatment or the like to remove impurities and achieve a higher purity and discoloration (removal of the coloring caused by the impurities). It is also possible to conduct purification by a known method such as crystallization (recrystallization), sublimation purification, re-slurry treatment or the like to obtain an intended product of high purity. Incidentally, the purity of 4,9-dihalogenated diamantane compound can be measured by GC.

Here, the re-slurry treatment refers, in the case of the step (i) of the present invention, to a treatment of adding, to the crude product of intended 4,9-dihalogenated diamantane compound, an organic solvent capable of selectively dissolving the impurities contained in the crude product, to prepare a slurry and then filtering the slurry, followed by drying, to obtain a 4,9-dihalogenated diamantane compound of high purity.

### Step (ii)

In the step (ii) of the production method of the present invention, the 4,9-dihalogenated diamantane compound obtained in the step (i) is hydrolyzed to obtain a 4,9-diamantanediol compound.

The hydrolysis reaction in the step (ii) is a reaction between 4,9-dihalogenated diamantane compound and water in the presence of a water-soluble organic solvent and a carboxylic acid salt.

As the water-soluble organic solvent used in the present invention, a known organic solvent miscible with water at normal temperature can be used with no restriction. Specifically, there can be mentioned methanol, ethanol, 2-propanol, 2-methoxyethanol, 2-ethoxyethanol, acetone, acetonitrile, N,N-dimethylformamide, formamide, triethylamine, pyridine, N,N,N',N'-tetramethylethylenediamine, etc. N,N-dimethylformamide is particularly preferred for the high reactivity and the low cost. In the present invention, the use amount of the water-soluble organic solvent is not particularly restricted; however, the amount is preferably 0.5 to 100 moles, more preferably 1 to 50 moles relative to 1 mole of the 4,9-dihalogenated diamantane compound used, in view of good yield and high reactivity.

The water used in the step (ii) has a function of hydrolyzing the 4,9-dihalogenated diamantane compound used as a raw material. The use amount thereof is preferably 5 to 500 moles, more preferably 20 to 400 moles relative to 1 mole of the 4,9-dihalogenated diamantane compound used, in view of high reactivity and recovery rate.

The carboxylic acid salt used in the step (ii) generates, in water, a cation for removing the halogen of 4,9-dihalogenated diamantane compound. In the step (ii), the cation derived from the carboxylic acid salt absorbs the hydrogen halide generated when the 4,9-dihalogenated diamantane compound is reacted with water to become a 4,9-diamantanediol compound. As the carboxylic acid salt, a known carboxylic acid salt can be used with no restriction. An alkali metal salt or an alkaline earth metal salt is preferred. Specifically, there can be mentioned alkali metal formates such as lithium formate, sodium formate, potassium formate and the like; alkaline earth metal formates such as magnesium formate, calcium formate, barium formate and the like; alkali metal acetates such as lithium acetate, sodium acetate, potassium acetate and the like; alkaline earth metal acetates such as magnesium acetate, calcium acetate, barium acetate and the like; and so forth.

Lithium acetate, sodium acetate and potassium acetate are preferred from the good availability and the high reactivity. These carboxylic acid salts may be used in admixture of two or more kinds.

There is no particular restriction as to the use amount of the carboxylic acid salt. However, the use amount is preferably 0.5 to 10 moles, more preferably 1 to 5 moles relative to 1 mole of the 4,9-dihalogenated diamantane compound used, from the standpoints of good yield and high reactivity.

In the step (ii), there is no particular restriction as to the temperature of hydrolysis reaction. However, too low a temperature makes slow the progress of the reaction and too high a temperature results in inferior operability. Therefore, the reaction temperature is preferably 100 to 200°C, more preferably 120 to 180°C.

There is no particular restriction as to the reaction time. However, ordinarily, a sufficient conversion can be obtained with 3 to 48 hours, and 3 to 24 hours is preferred.

The hydrolysis reaction is ordinarily conducted preferably in a closed system (e.g. an autoclave) so that the vaporization of solvent is prevented and the intended reaction temperature can be reached easily. In that case, the pressure is an applied (by about 0.2 to 0.8 MPa) pressure (the reaction pressure differs depending upon the reaction temperature used).

The thus-obtained 4,9-diamantanediol compound is represented by the following formula (6). 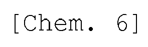 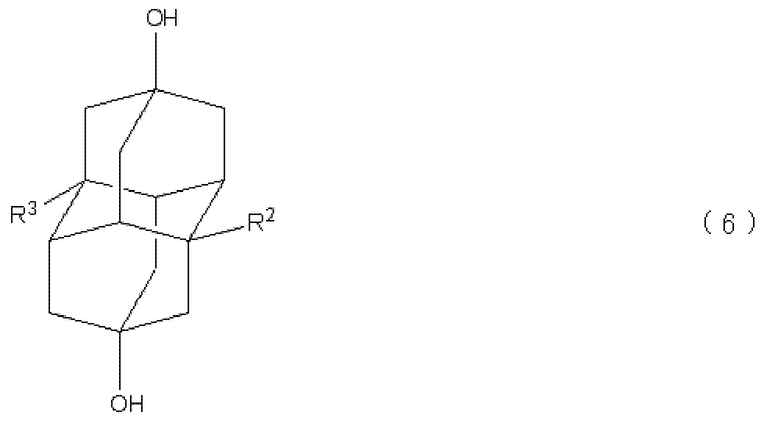 {wherein R² and R³ have each the same definition as for the R² and R³ of the formula (1) }.

The chemical structure of the compound of the formula (6) depends on the chemical structure of the compound of the formula (2) used. As specific examples of the 4,9-diamantanediol compound, there can be mentioned 4,9-diamantanediol, 1-methyl-4,9-diamantanediol, 1,6-dimethyl-4,9-diamantanediol, 1-ethyl-4,9-diamantanediol, 1,6-diethyl-4,9-diamantanediol, etc.

As to the method for isolating the 4,9-diamantanediol compound formed in the above reaction, there is no particular restriction. However, the isolation can be conducted, for example, by the following method. The method is a case using an autoclave as a reactor.

Firstly, after the reaction, the reaction mixture is cooled to room temperature or lower and the pressure inside the autoclave is returned to normal pressure. The reaction mixture inside the autoclave, containing the precipitated solid is taken out and subjected to a means such as filtration, centrifugation or the like for separation to a liquid and a solid. The solid obtained contains a 4,9-diadamantanediol compound and an alkali metal salt or an alkaline earth metal salt, derived from a carboxylic acid salt. The solid is washed with water to remove the salt, whereby is obtained a crude product of 4,9-diamantanediol compound (the conversion based on raw material 4,9-dichlorodiamantane compound is 95% or more).

The 4,9-diamantanediol compound obtained by the above isolation method can be sufficiently used per se as a raw material for the next step (iii). However, an adsorption treatment such as active carbon treatment, silica treatment, alumina treatment or the like may be conducted as necessary for removal of impurities and consequent higher purity and discoloration (removal of the coloring caused by impurities). Besides such a treatment, purification may be conducted by a known method such as crystallization (recrystallization), sublimation purification, re-slurry treatment or the like, to obtain an intended product of high purity. Incidentally, purity can be confirmed by GC analysis.

Here, the re-slurry indicates, in the case of the step (ii) of the present invention, a method of adding, to the crude product of 4,9-diamantanediol compound, an organic solvent capable of selectively dissolving the impurities contained in the crude product, to obtain a slurry, filtering the slurry, and drying the solid separated by the filtration, to obtain a 4,9-diamantanediol compound of high purity.

### Step (iii)

In the step (iii) of the production method of the present invention, the 4,9-diamantanediol compound obtained in the step (ii) is esterified in a mixture of a polymerizable unsaturated carboxylic acid represented by the formula (3) and a polymerizable unsaturated carboxylic acid anhydride represented by the formula (4) in the presence of a polymerization inhibitor and an acid catalyst, to obtain a crude product of a polymerizable hydroxydiamantyl ester compound represented by the formula (1).

By using, as an esterifying agent, a mixture of a polymerizable unsaturated carboxylic acid and a polymerizable unsaturated carboxylic acid anhydride, a crude product of polymerizable hydroxydiamantyl ester compound, of low coloring is obtained even when the esterification reaction is conducted at a relatively high temperature. There is no such advantage when an acid chloride is used.

When the reaction of the step (iii) is conducted in the absence of polymerization inhibitor, the intended polymerizable hydroxydiamantyl ester compound and the polymerizable unsaturated carboxylic acid anhydride cause polymerization and incurs striking formation of high-molecular impurity components having molecular weights of about 300 to 5,000 (hereinafter, these impurity components are referred to as "oligomer impurities") and polymer impurities (impurity components having higher molecular weights than the oligomer impurities). In this case, a high-degree purification operation is required in order to obtain a product of high purity, resulting in a significant reduction in yield, associated with the purification operation.

Incidentally, measurement of the content of oligomer impurities or polymer impurities can be conducted by gel permeation chromatography (hereinafter referred to simply as GPC) .

In the present invention, there is used, as the polymerizable unsaturated carboxylic acid, a polymerizable unsaturated carboxylic acid represented by the following formula (3) 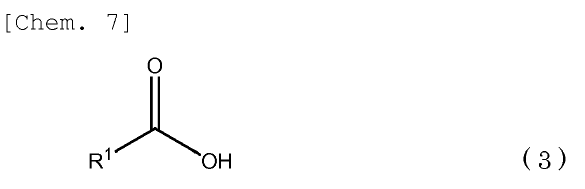 (wherein R¹ is a polymerizable unsaturated hydrocarbon group which may have a substituent group), from the standpoint of the usefulness of the polymerizable hydroxydiamantyl ester compound obtained.

The R¹, i.e. the polymerizable unsaturated hydrocarbon group which may have a substituent group, may be any of straight chain, branched chain and cyclic polymerizable unsaturated hydrocarbon groups. As specific examples thereof, there can be mentioned vinyl group, iso-propenyl group, allyl group, 1-propenyl group, 3-butenyl group, 3-methyl-3-butenyl group, 4-pentenyl group and 1,3-butadienyl group.

As the substituent group possessed by the R¹, there can be mentioned cyano group; halogen atoms such as fluorine, chlorine, bromine, iodine and the like; alkoxy groups of 1 to 5 carbon atoms such as methoxy group, ethoxy group and the like; and so forth.

As specific examples of the polymerizable unsaturated carboxylic acid preferably usable in the step (iii), there can be mentioned acrylic acid, methacrylic acid, vinylacetic acid, crotonic acid, 4-pentenoic acid, 4-mehtyl-4-pentenoic acid, 5-hexenoic acid, and 2,4-pentadienoic acid. Of these compounds, acrylic acid or methacrylic acid is particularly preferred from the standpoint of the usefulness of product obtained.

In the present invention, there is used, as the polymerizable unsaturated carboxylic acid anhydride, a polymerizable unsaturated carboxylic acid anhydride represented by the following formula (4) 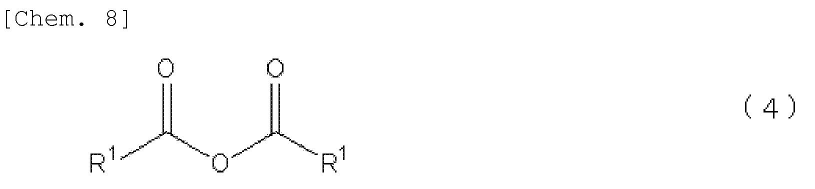 (wherein R¹ is a polymerizable unsaturated hydrocarbon group which may have a substituent group), from the standpoint of the usefulness of the polymerizable hydroxydiamantyl ester compound obtained.

The R¹, i.e. the polymerizable unsaturated hydrocarbon group which may have a substituent group, may be any of straight chain, branched chain and cyclic polymerizable unsaturated hydrocarbon groups. As specific examples thereof, there can be mentioned vinyl group, iso-propenyl group, allyl group, 1-propenyl group, 3-butenyl group, 3-methyl-3-butenyl group, 4-pentenyl group and 1,3-butadienyl group.

As the substituent group possessed by the R¹, there can be mentioned cyano group; halogen atoms such as fluorine, chlorine, bromine, iodine and the like; alkoxy groups of 1 to 5 carbon atoms such as methoxy group, ethoxy group and the like; and so forth.

The two R¹S possessed by the compound represented by the formula (4) may be different from each other but are preferably the same. Further, these R¹S may be the same as or different from the R¹ possessed by the polymerizable unsaturated carboxylic acid.

In the present invention, a mixture of the above-mentioned polymerizable unsaturated carboxylic acid and polymerizable unsaturated carboxylic acid anhydride is used in the esterification reaction. In the esterification of the present invention, it is not certain which of the polymerizable unsaturated carboxylic acid and the polymerizable unsaturated carboxylic acid anhydride functions as an esterifying agent. However, it is considered that the polymerizable unsaturated carboxylic acid acts with the solvent and dissolves mainly the 4,9-diamantanediol compound represented by the formula (6) and, in the solution, the 4,9-diamantanediol compound reacts with the polymerizable unsaturated carboxylic acid anhydride. In this case, either of the two R¹S possessed by the polymerizable unsaturated carboxylic acid anhydride represented by the formula (4) is introduced into an intended product (a polymerizable hydroxydiamantyl ester compound).

However, it is considered that the polymerizable unsaturated carboxylic acid acts not only as a solvent for 4,9-diadamantanediol compound but also as an esterifying agent. In this case, it is considered that the polymerizable unsaturated carboxylic acid gives rise to an esterification reaction with the 4,9-diadamantanediol compound to form an intended polymerizable hydroxydiamantyl ester compound. At that time, the polymerizable unsaturated carboxylic acid anhydride acts as a dehydrating agent, whereby the anhydride is converted into a polymerizable unsaturated carboxylic acid. In this case, the R¹ possessed by the polymerizable unsaturated carboxylic acid represented by the formula (3) is introduced into an intended product (a polymerizable hydroxydiamantyl ester compound) .

The polymerizable unsaturated carboxylic acid represented by the formula (3) and the polymerizable unsaturated carboxylic acid anhydride represented by the formula (4) may cause an exchange reaction.

Owing to occurring various reactions mentioned above, when the R¹S possessed by the polymerizable unsaturated carboxylic acid and the polymerizable unsaturated carboxylic acid anhydride are different from each other, it may be a case that the polymerizable hydroxydiamantyl ester compound obtained may be two or three kinds of compounds having different polymerizable unsaturated hydrocarbon groups.

For these reasons, in order to obtain a single, polymerizable hydroxydiamantyl ester compound, it is preferred to use a polymerizable unsaturated carboxylic acid and a polymerizable unsaturated carboxylic acid anhydride, both having the same R¹. For example, when acrylic acid is used as the polymerizable unsaturated carboxylic acid, acrylic acid anhydride is used as the polymerizable unsaturated carboxylic acid anhydride. Similarly, when methacrylic acid is used as the polymerizable unsaturated carboxylic acid, methacrylic acid anhydride is used as the polymerizable unsaturated carboxylic acid anhydride. When it is not necessary to obtain a single compound, there can be used a combination of a polymerizable unsaturated carboxylic acid and a polymerizable unsaturated carboxylic acid anhydride, each having a different R¹.

Next, explanation is made on the mixing ratio of the polymerizable unsaturated carboxylic acid and the polymerizable unsaturated carboxylic acid anhydride. When the amount of polymerizable unsaturated carboxylic acid is too small as compared to the amount of 4,9-diamantanediol (raw material), the progress of reaction is extremely slow; when the amount is too large, the post-treatment is complicated. When the amount of polymerizable unsaturated carboxylic acid anhydride is too small as compared to the amount of 4,9-diamantanediol (raw material), the progress of reaction is extremely slow or the reaction stops in a state that the raw material remains in a large amount; when the amount is too large, a di-ester compound (a by-product) is formed in a large amount. Therefore, the mixing ratio of the polymerizable unsaturated carboxylic acid and the polymerizable unsaturated carboxylic acid anhydride is as follows. The amount of polymerizable unsaturated carboxylic acid is 3 to 200 moles and the amount of polymerizable unsaturated carboxylic acid anhydride is 1 to 3 moles relative to 1 mole of 4,9-diadamantanediol (raw material); preferably, the amount of polymerizable unsaturated carboxylic acid is 3 to 100 moles and the amount of polymerizable unsaturated carboxylic acid anhydride is 1 to 3 moles; more preferably, the amount of polymerizable unsaturated carboxylic acid is 3 to 70 moles and the amount of polymerizable unsaturated carboxylic acid anhydride is 1 to 2 moles; particularly preferably, the amount of polymerizable unsaturated carboxylic acid is 5 to 50 moles and the amount of polymerizable unsaturated carboxylic acid anhydride is 1 to 2 moles; most preferably, the amount of polymerizable unsaturated carboxylic acid is 5 to 40 moles and the amount of polymerizable unsaturated carboxylic acid anhydride is 1 to 2 moles.

The esterification reaction of 4,9-diamantanediol compound in the mixture of the polymerizable unsaturated carboxylic acid and the polymerizable unsaturated carboxylic acid anhydride can be conducted in the presence or absence of an organic solvent. However, since the 4,9-diamatanediol has low solubility in organic solvent, a reaction using an organic solvent may cause problems such as extremely slow progress, formation of large amount of di-ester compound, and the like. Hence, the above reaction of the present invention is conducted preferably in a state that an organic solvent is present in the reaction mixture in an amount of 10% by mass or less, preferably 5% by mass or less, and is conducted particularly preferably in the absence of an organic solvent.

In the step (iii), the esterification reaction of 4,9-diamantanediol compound is conducted in the presence of an acid catalyst.

As the acid catalyst, there can be used a known acid which is known to function as an acid catalyst in esterification, such as inorganic acid (e.g. sulfuric acid), sulfonic acid (e.g. methanesufonic acid, benzenesulfonic acid or p-toluenesulfonic acid), acetic acid (e.g. trifluoroacetic acid, trichloroacetic acid or tribromoacetic acid) or the like. Of these acid catalysts, at least one kind of acid selected from sulfuric acid, methanesulfonic acid, benzenesulfonic acid and p-toluenesulfonic acid is preferred for the reasons of high effect for suppression of by-product formation and low cost.

As to the use amount of the acid catalyst, there is no particular restriction. However, too small an amount results in an extremely low reaction rate. Too large an amount results in a low yield caused by side reaction. Therefore, the use amount of the acid catalyst is preferably 0.01 to 10% by mass, more preferably 0.1 to 5% by mass based on the mass of the 4,9-diamantanediol compound used.

In general, in the esterification reaction of a polymerizable unsaturated carboxylic acid using an acid catalyst, formation of oligomer impurities and polymer impurities is active. As described previously, in the production method of the present invention as well, the esterification need be conducted in the presence of a polymerization inhibitor.

As the polymerization inhibitor, an effective polymerization inhibitor may be appropriately selected from known polymerization inhibitors. As examples of the polymerization inhibitor preferably usable in the present invention, there can be mentioned phenol type compounds such as hydroquinone, hydroquinone monomethyl ether, 2,6-di-tert-butyl-4-methylphenol and the like; quinone type compounds such as benzoquinone, naphthoquinone and the like; amine type compounds such as phenothiazine, aniline and the like; nitroxy radical type compounds such as 2,2,6,6-tetramethylpiperizine-N-oxyl and the like; copper compounds such as copper dithiocarbamate and the like; sulfur compounds; phosphorus compounds; oxygen; "Sumilizer-GM", "Sumilizer-TP-D" and "Sumilizer-WX-R" (trade names, products of Sumitomo Chemical Company, Ltd.); and so forth. These polymerization inhibitors may be used in combination of two or more kinds.

The use amount of the polymerization inhibitor is preferably 0.01 to 10% by mass, more preferably 0.01 to 5% by mass based on the mass of the 4,9-diamantanediol compound used, from the standpoints of the inhibition effect for polymerization and the prevention of excessive use.

As to the temperature of the esterification reaction in the step (iii), there is no particular restriction. However, the temperature is preferably 10 to 150°C, more preferably 30 to 120°C in order to suppress the formation of oligomer impurities and complete the reaction in a relatively short time. The time of the reaction may be determined appropriately depending upon other reaction conditions; however, a sufficient conversion can be obtained ordinarily in a reaction time of 0.5 to 24 hours. The reaction time is preferably determined while confirming the progress of the reaction.

The polymerizable hydroxydiamantyl ester compound obtained in the step (iii) is presented by the following formula (1) 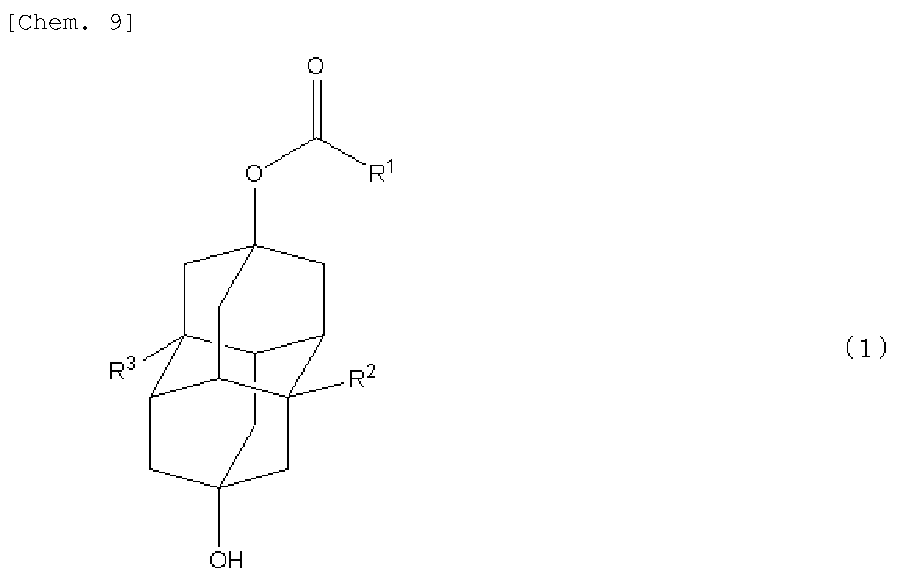 (wherein R¹ is a polymerizable unsaturated hydrocarbon group which may have a substituent, and R² and R³ are each independently a hydrogen atom or an alkyl group of 1 to 5 carbon atoms).

As the polymerizable hydroxydiamantyl ester compound represented by the formula (1), produced via the steps (i) to (iii), there are preferred 9-hydroxy-4-diamantyl acrylate, 9-hydroxy-4-diamantyl methacrylate, 9-hydroxy-1-methyl-4-diamantyl acrylate, 9-hydroxy-1-methyl-4-diamantyl methacrylate, 9-hydroxy-1,6-dimethyl-4-diamantyl acrylate, 9-hydroxy-1,6-dimethyl-4-diamantyl methacrylate, 9-hydroxy-1,6-diethyl-4-diamantyl acrylate, 9-hydroxy-1,6-diethyl-4-diamantyl methacrylate, etc. Of these, 9-hydroxy-4-diamantyl acrylate and 9-hydroxy-4-diamantyl methacrylate are particularly preferred for their usefulness.

As to the method for separating the polymerizable hydroxydiamantyl ester compound (intended product) from the post-esterification reaction mixture obtained in the step (iii), there is no particular restriction. However, the following method, for example, can be mentioned.

That is, first, an organic solvent is added to the reaction mixture to migrate the intended product into the organic solvent layer; then, an aqueous basic solution is added to decompose the polymerizable unsaturated carboxylic acid anhydride remaining in the reaction mixture, into an unsaturated carboxylic acid and also neutralize the unsaturated carboxylic acid. Thereafter, the organic solvent layer is separated and is washed with water several times until the water layer becomes neutral. Next, as necessary, there is conducted an adsorption treatment such as active carbon treatment, silica treatment, alumina treatment or the like, after which the solvent is distilled off, followed by drying, to obtain a crude intended product. This crude product contains a polymerizable hydroxydiamantyl ester compound (an intended product) ordinarily in an amount of 60% or more in terms of GC purity.

Incidentally, in the adsorption treatment, the extraction solvent may be exchanged with other solvent. The adsorption treatment using exchanged solvent (other solvent) is conducted when the adsorption treatment using the extraction solvent provides no sufficient purification. Ordinarily, the adsorption treatment using the extraction solvent is preferred because the time of step (iii) is shorter. The solvent used in the exchange varies from relatively low-polarity solvents of aliphatic hydrocarbons such as heptane and the like, to relatively high-polarity solvents of methanol, acetonitrile, water, etc., and a known solvent can be used with no restriction.

The adsorption treatment allows for efficient removal of colored components contained in an extremely small amount. The crude product obtained is subjected to further purification by a known method such as crystallization (recrystallization), vacuum distillation, steam distillation, sublimation purification or the like, whereby an intended product of high purity can be obtained.

As to the organic solvent used as an extraction solvent in the above-mentioned separation of polymerizable hydroxydiamantyl ester compound, there is no particular restriction. However, from the standpoints of the easiness of concentration and the easiness of extraction operation, there are preferred halogenated aliphatic hydrocarbons such as methylene chloride, chloroform, carbon tetrachloride and the like; aliphatic hydrocarbons such as hexane, heptane, octane and the like; aromatic hydrocarbons such as toluene, xylene, chlorobenzene, bromobenzene and the like; ethers such as diethyl ether, di-isopropyl ether, di-n-butyl ether, di-tert-butyl ether and the like; ketones such as methyl ethyl ketone, methyl isobutyl ketone and the like; esters such as methyl acetate, ethyl acetate, propyl acetate, isopropyl acetate, butyl acetate, isobutyl acetate and the like; and so forth.

Of these, halogenated aliphatic hydrocarbons and esters are particularly preferred, and methylene chloride is most preferred. These organic solvents are preferred because they show especially high solubilities to polymerizable hydroxydiamantyl ester compound.

As to the use amount of the organic solvent, there is no particular restriction. However, the use amount is preferably 0.1 to 100 parts by mass, particularly preferably 0.5 to 50 parts by mass relative to 1 part by mass of 4,9-diamantanediol compound (raw material), from the standpoints of the solubility for polymerizable hydroxydiamantyl ester compound and the easiness of concentration.

When the intended product is a solid at normal temperature, it is preferred that, after the adsorption treatment, the crude product obtained is subjected to a crystallization treatment for further purification.

Here, the crude polymerizable hydroxydiamantyl ester compound before crystallization contains, as a by-product, ordinarily about 10 to 30% (in terms of peak area % by GC) of a 4,9-di-ester compound (a compound in which each of the two hydroxyl groups of 4- and 9-positions is changed in a state of a polymerizable unsaturated carboxylic acid ester).

The reason why the di-ester compound is by-produced, is explained below in comparison with the case of similar adamantane derivative.

When a polymerizable hydroxyadamantyl ester compound is produced using a 1,3-adamantanediol as a raw material, the positions of the two hydroxyl groups in the raw material molecule are relatively near from each other; therefore, it is considered that there is light steric hindrance in introduction of second ester group and that the introduction of first ester group reduces the nucleophilicity of residual hydroxyl group. That is, owing to the steric and electronic reasons, the rate of esterification of second hydroxyl group after the esterification of first hydroxyl group is relatively low. As result, an intended, mono-esterified product can be obtained at a high selectively.

Meanwhile, when a polymerizable hydroxydiamantyl ester compound is produced using a 4,9-diamantanediol as a raw material, the positions of the two hydroxyl groups in the raw material molecule are far apart from each other; therefore, it is considered that the steric hindrance in introduction of second ester group is small and that the electronic influence caused by introduction of first ester group is small as well. Thus, the steric and electronic environments differ from the case of adamantane derivative and this is considered to make difficult the achievement of high selectivity in the reaction step. Therefore, in a reaction in high conversion, about 10 to 30% of a 4,9-di-ester compound is inevitably contained in the polymerizable hydroxydiamantyl ester compound before crystallization.

However, the 4,9-di-ester compound is easily separated by the crystallization employed in the present invention, and an intended product of high purity can be obtained.

As the crystallization solvent, a solvent containing at least an aromatic hydrocarbon is preferred. Use of such a solvent allows for efficient separation of intended polymerizable hydroxydiamantyl ester compound and by-produced, 4,9-di-ester compound.

As to the aromatic hydrocarbon used as the crystallization solvent, there is no particular restriction as long as it can dissolve the polymerizable hydroxydiamantyl ester compound at room temperature or by heating. However, there are preferred, from the good availability, benzene, toluene, ethylbenzene, n-propylbenzene, isopropylbenzene, n-butylbenzene, sec-butylbenzene, tert-butylbenzene, xylene (o-xylene, m-xylene or p-xylene), chlorobenzene, bromobenzene, dichlorobenzene, dibromobenzene, etc. In view of the high purification degree of the polymerizable hydroxydiamantyl ester compound to be obtained, benzene and toluene are preferred and toluene is more preferred. These aromatic hydrocarbons may be used in admixture of two or more kinds.

Other organic solvent may be used in combination with the aromatic hydrocarbon. The organic solvent usable in combination with the aromatic hydrocarbon may be any solvent capable of dissolving the polymerizable hydroxydiamantyl ester compound in combination with the aromatic hydrocarbon, at room temperature or by heating. As examples of such an organic solvent, there can be mentioned, for the good availability, halogenated aliphatic hydrocarbons such as methylene chloride, chloroform, carbon tetrachloride and the like; aliphatic hydrocarbons such as hexane, heptane, octane and the like; ethers such as diethyl ether, di-isopropyl ether, di-n-butyl ether, di-tert-butyl ether and the like; ketones such as acetone, methyl ethyl ketone, methyl isobutyl ketone and the like; esters such as methyl acetate, ethyl acetate, propyl acetate, isopropyl acetate, butyl acetate, isobutyl acetate and the like; and alcohols such as methanol, ethanol, isopropyl alcohol, n-propyl alcohol, n-butanol, n-octanol and the like.

Of these organic solvents, aliphatic hydrocarbons such as hexane, heptane, octane and the like are preferred because they can purify the intended, polymerizable hydroxydiamantyl ester compound in a high purity. These organic solvents other than aromatic hydrocarbon may be used in combination of two or more kinds.

As to the use amount of the aromatic hydrocarbon used as a crystallization solvent, there is no particular restriction. However, the use amount is preferably 0.1 to 50 parts by mass, more preferably 0.5 to 20 parts by mass, particularly preferably 1 to 10 parts by mass relative to 1 part by mass of 4,9-diamantanediol compound (raw material).

When the crystallization is conducted using a combination of an aromatic hydrocarbon and other organic solvent, the use amount of the other organic solvent is not particularly restricted. However, the use amount is preferably 0.01 to 30 parts by mass, more preferably 0.05 to 20 parts by mass, particularly preferably 0.1 to 10 parts by mass relative to 1 part by mass of 4,9-diamantanediol compound (raw material).

As to the proportions of the aromatic hydrocarbon and other solvent used in combination in the crystallization, there is no particular restriction. However, the proportion of other solvent is preferably small in view of the purity of purified product. The aromatic hydrocarbon (mass) : other solvent (mass) is preferably 1 : 1 or less, more preferably 1 : 0.75 or less, and most preferably 1 : 0.5 or less.

As to the temperature of crystallization, there is no particular restriction. However, the temperature is preferably 20°C or lower, more preferably 10°C or lower, from the view point of the amount of intended product obtained. There is no particular restriction, either, as to the time of crystallization. However, an intended product can be obtained at a high yield and at a high purity ordinarily in 1 to 24 hours.

The following is an example of the crystallization operation.

First, toluene is added to a crude product. The mixture is heated once to about 30 to 60°C (the temperature differs depending upon the kind of the product) to obtain a uniform solution. Then, the solution is cooled to 10°C or lower to separate out crystals. The mixture is aged in that state for about 1 to 2 hours. The crystals formed are recovered by filtration or the like, whereby a polymerizable hydroxydiamantyl ester compound ordinarily having a purity of 97% by mass or more can be obtained.

### Examples

The present invention is described more specifically below by way of Examples. However, the present invention is in no way restricted by these Examples.

### Example 1

### Step (i)

In a 2000-ml, four-necked flask was placed, in a nitrogen current, 240 g (two times the mass of raw material diamantane) of concentrated sulfuric acid and 9.05 g (0.0637 mole, 0.1 time the mole of raw material diamantane) of sodium sulfate. Then, 120 g (0.637 mole) of diamantane was placed. The mixture of suspension state was heated to about 30°C. Thereto was slowly added 148.4 g (1.274 moles, 2 times the mole of raw material diamantane) of chlorosulfonic acid so that there was no bumping of the mixture. The mixture was stirred at 30°C for 3 hours. The reaction mixture after 3 hours of stirring was in a suspension state. At this time, GC analysis was conducted. As a result, the amount of remaining raw material diamantane was 70% (this indicates a peak area ratio of GC; the same applies hereinafter), the amount of monochlorodiamantane formed was 3%, the amount of dichlorodiamantane formed was 24%, and the amount of trichlorodiamantane formed was 3%; and the reaction was in stop.

Thereto was added 222.7 g [1.912 moles, 3 times (5 times in total) the mole of raw material diamantane]. The mixture was stirred at 30°C for 15 hours (the reaction time was 18 hours in total). After the 15 hours, GC analysis was conducted. As a result, the amount of remaining raw material diamantane was 1%, the amount of monochlorodiamantane formed was 5%, the amount of dichlorodiamantane formed was 80%, and the amount of trichlorodiamantane formed was 14%. The reaction mixture was cooled to 10°C. Thereto was dropwise added 262 g of water while the temperature of the reaction mixture was maintained so as not to exceed 30°c. 840 g of methylene chloride was added. The mixture was stirred and then allowed to stand. Phase separation occurred. The lower aqueous sulfuric acid solution layer was removed. Then, the organic layer was washed with 240 g of a 10% aqueous sodium hydroxide solution twice, 300 g of a 7% aqueous sodium sulfate solution once, and 120 g of a 7% aqueous sodium sulfate solution twice, whereby the pH of the organic layer became neutral. The organic layer was concentrated under reduced pressure and dried to obtain 155 g of a cream-colored solid containing 80% of 4,9-dichlorodiamantane. The yield was 74% based on diamantane.

### Step (ii)

In a 1000-ml, glass-made autoclave were placed 155 g (0.600 mole converting that the purity was 100%) of the 4,9-dichlorodiamantane obtained in the step (i), 98.5 g (1.20 moles, 2 times the mole of 4,9-dichlorodiamantane) of sodium acetate, 87.7 g (1.20 moles, 2 times the mole of 4,9-dichlorodiamantane) of N,N-dimethylformamide, and 386.3 g (21.5 moles, 35.8 times the mole of 4,9-dichlorodiamantane) of ion exchanged water. The mixture was heated to 150°C in a closed state. With the temperature 150°C and 0.4 MPa being maintained, stirring was conducted for 21 hours. After 26 hours, the mixture was cooled to 5°C. The solid, which separated out in the autoclave, was separated by filtration and washed with 155 g of ion exchange water twice, to obtain 150 g of a light brown solid.

To the light brown solid was added, as a re-slurry solvent, 450 g [3 times the mass of the light brown solid (crude 4,9-diamantanediol) ] of ethyl acetate. The mixture was stirred at 70°C for 1.5 hours. At this time, the 4,9-diamantanediol was not in complete dissolution in ethyl acetate and was in a suspended state. The mixture was cooled to 5°C, stirred for 2 hours, and subjected to aging. A solid was filtered and dried to obtain 86 g (yield based on diamantane: 61%) of a white solid. This solid was analyzed by GC. As result, the content of 4,9-diamantanediol was 99% in terms of GC purity.

### Step (iii)

The inside of a 2000-ml, four-necked flask was purged with nitrogen sufficiently, after which nitrogen gas was blown into continuously. Into the four-necked flask were added 40 g (0.182 mole) of the 4,9-diamantanediol obtained in the step (ii), 0.20 g (0.5% relative to the mass of 4,9-diamantanediol) of phenothiazine (polymerization inhibitor), and 400 g (4.65 moles, 10 times the mass of 4,9-diamantanediol) of methacrylic acid (organic solvent). The mixture was heated to 90°C. Thereto was added 0.4 g (1% relative to the mass of 4,9-diamantanediol) of concentrated sulfuric acid (acid catalyst). The mixture changed from a colorless state to a slightly reddish state and was in a suspension state.

Thereto was dropwise added 29.23 g (0.182 mole, equal to the mole of 4,9-diamantanediol) of methacrylic acid anhydride (polymerizable unsaturated carboxylic acid anhydride). The mixture was stirred at 90°C for 5 hours. Then GC analysis was conducted. The amount of remaining diamantanediol (raw material) was 8%, the amount of intended 9-hydroxy-4-diamantyl methacrylate formed was 71%, and the amount of 4,9-di-ester compound (by-product) was 21%. The reaction mixture was a uniform solution.

Then, the reaction mixture was cooled to room temperature. Thereto was added 400 g of methylene chloride (extraction solvent), followed by cooling to 5°C. Thereto was dropwise added 967.3 g of a 20 mass % aqueous sodium hydroxide solution while the temperature of the reaction mixture was maintained so as not to exceed 25°C. The organic layer and the aqueous layer were separated, and the organic layer was washed with 400 g of ion exchange water 4 times. The pHs of the organic layer and the aqueous layer after the washing were neutral.

To the organic layer was added 4 g (0.1 time the mass of 4,9-diamantanediol) of active carbon, followed by stirring at room temperature for 1 hour. Then, the active carbon was removed by filtration to obtain a nearly transparent solution of 9-hydroxy-4-diamantyl methacrylate. Then, 0.04 g (0.1% relative to the mass of 4,9-diamantanediol) of hydroquinone monomethyl ether was added, followed by distillation under reduced pressure, to obtain crude 9-hydroxy-4-diamantyl methacrylate.

### Crystallization step

To the crude 9-hydroxy-4-diamantyl methacrylate obtained was added 240g (6 times the mass of 4,9-diamantanediol) of toluene (aromatic hydrocarbon) as a crystallization solvent. The mixture was heated to 53°C for dissolution. The solution was cooled slowly to 2°c to separate out crystals. Aging was conducted at 2°C for 1 hour. Then, the crystals were collected by filtration and dried to obtain 21 g (yield: 40%) of a white solid. The solid was analyzed by GC and GPC. As a result, the content of 9-hydroxy-4-diamantyl methacrylate was 99% in terms of GC purity and the polystyrene-represented content of oligomer impurities was 0.1%.

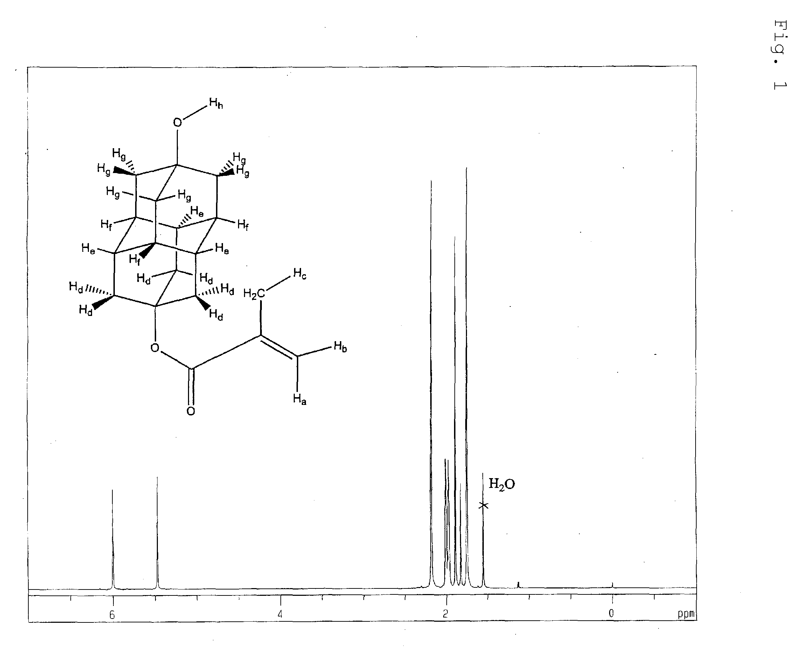

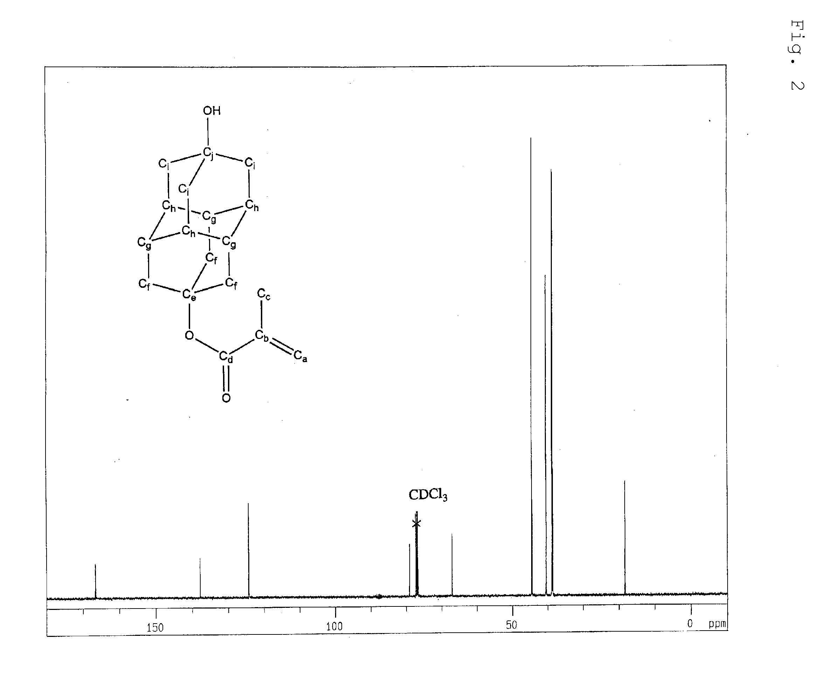

The proton NMR (¹H-NMR) spectrum of the obtained 9-hydroxy-4-diamantyl methacrylate is shown in Fig. 1, and its ¹³C-NMR spectrum is shown in Fig. 2.
Mass spectrum (EI method): molecular weight 288 (M⁺)
¹H-NMR [TMS (standard substance), in CDCl₃] :
δ1.75 (H_{g}, d, 6H), δ1.82 (Hₕ, s, 1H), δ1.89 (H_{c}, m, 3H), δ1.97 ∼ 2.00 (He, H_{f}, m, 6H), δ2.18 (H_{d}, d, 6H), δ 5.48 (H_{b}, m, 1H), δ 6.00 (Hₐ, m, 1H)
¹³C-NMR (in CDCl₃) :
δ18.2 (C_{c}), δ 388.6, 38.8 (C_{g}, Cₕ), δ 40.5 (C_{f}), δ 44.5 (Cᵢ), δ 67.0 (Cⱼ), δ79.1 (Cₑ), δ124.4 (Cₐ), δ137.9 (C_{b}), δ 166.6 (Cₐ)

### Examples 2 to 3

An operation was conducted in the same manner as in Example 1 except that the crystallization solvent used in the crystallization step was changed to one shown in Table 1. The results are shown in Table 1.
[Table 1]

**Table 1**

| Example | Crystallization solvent | | | | Polymerizable hydroxydiamantyl ester compound | | | |
|---|---|---|---|---|---|---|---|---|
| | Aromatic hydrocarbon | | Other solvent except aromatic hydrocarbon | | | | | |
| | Kind | Amount | Kind | Amount | Yield (g) | Yield (%) | GC purity (%) | Oligomer amount (%) |
| 2 | Toluene | 6 mass times | Heptane | 2 mass times | 23 | 44 | 98 | 0.2 |
| 3 | Toluene | 6 mass times | Heptane | 1 mass time | 22 | 42 | 99 | 0.1 |

### Examples 4 to 6

An operation was conducted in the same manner as in Example 1 except that, in the step (iii), the acid catalyst was changed to one shown in Table 2. The results are shown in Table 2.
[Table 2]

**Table 2**

| Example | Acid catalyst | Polymerizable hydroxydiamantyl ester compound | | | |
|---|---|---|---|---|---|
| | | Yield (g) | Yield (%) | GC purity (%) | Oligomer amount (%) |
| 4 | Methanesulfonic acid | 21 | 40 | 99 | 0.1 |
| 5 | Benzenesulfonic acid | 21 | 40 | 99 | 0.1 |
| 6 | p-Toluenesulfonic acid | 20 | 38 | 99 | 0.1 |

### Example 7

The inside of a 1000-ml, four-necked flask was purged with nitrogen sufficiently, after which nitrogen gas was blown into continuously. Into the four-necked flask were added 20 g (0.0908 mole) of the 4,9-diamantanediol obtained in the step (ii) of Example 1, 0.10 g (0.5% relative to the mass of 4,9-diamantanediol) of phenothiazine (polymerization inhibitor), 200 g (2.32 moles, 10 times the mass of 4,9-diamantanediol) of methacrylic acid (organic solvent), and 14.6 g (0.0908 mole, equal to the mole of 4,9-diamantandiol) of methacrylic acid anhydride (polymerizable unsaturated carboxylic acid anhydride). The mixture was heated to 90°C. Thereto was added 0.2 g (1% relative to the mass of 4,9-diamantanediol) of concentrated sulfuric acid (acid catalyst).

The resulting mixture of various components was changed from a colorless state to a slightly reddish state and was in a suspension state. The mixture was stirred at 90°C for 7 hours. Then, GC analysis was conducted. The amount of remaining diamantanediol (raw material) was 9%, the amount of intended 9-hydroxy-4-diamantyl methacrylate formed was 69%, and the amount of 4,9-di-ester compound (by-product) was 22%. The reaction mixture was a uniform solution. The reaction mixture was cooled to room temperature. Thereto was added 200 g of methylene chloride (extraction solvent), followed by cooling to 5°C. Thereto was dropwise added 483.6 g of a 20 mass % aqueous sodium hydroxide solution while the temperature of the reaction mixture was maintained so as not to exceed 25°C. The organic layer and aqueous layer were separated, and the organic layer was washed with 200 g of ion exchange water 4 times. The pHs of the organic layer and the aqueous layer after the washing were neutral.

To the solution was added 2 g (0.1 time the mass of 4,9-diamantanediol) of active carbon, followed by stirring at room temperature for 1 hour. Then, the active carbon was removed by filtration to obtain a nearly transparent solution of 9-hydroxy-4-diamantyl methacrylate. To the solution was added 0.02 g (0.1% relative to the mass of 4,9-diamantanediol) of hydroquinone monomethyl ether, followed by distillation under reduced pressure, to obtain crude 9-hydroxy-4-diamantyl methacrylate.

To the crude 9-hydroxy-4-diamantyl methacrylate obtained was added 120g (6 times the mass of 4,9-diamantanediol) of toluene (crystallization solvent). The mixture was heated to 53°C for dissolution. The solution was cooled slowly to 2°C to separate out crystals. Aging was conducted at 2°C for 1 hour. The crystals were collected by filtration and dried to obtain 10 g (yield: 38%) of a white solid. The solid was analyzed by GC and GPC. As a result, the content of 9-hydroxy-4-diamantyl methacrylate was 99% in terms of GC purity and the polystyrene-represented content of oligomer impurities was 0.1%.

### Examples 8 to 9

An operation was conducted in the same manner as in Example 7 except that the crystallization solvent was changed to one shown in Table 3. The results are shown in Table 3.
[Table 3]

**Table 3**

| Example | Crystallization solvent | | | | Polymerizable hydroxydiamantyl ester compound | | | |
|---|---|---|---|---|---|---|---|---|
| | Aromatic hydrocarbon | | Other solvent except aromatic hydrocarbon | | | | | |
| | Kind | Amount | Kind | Amount | Yield (g) | Yield (%) | GC purity (%) | Oligomer amount (%) |
| 8 | Toluene | 6 mass times | Heptane | 2 mass times | 11 | 42 | 98 | 0.2 |
| 9 | Toluene | 6 mass times | Heptane | 1 mass time | 10 | 38 | 99 | 0.1 |

### Example 10

The inside of a 1000-ml, four-necked flask was purged with nitrogen sufficiently, after which nitrogen gas was blown into continuously. Into the four-necked flask were added 20 g (0.0908 mole) of the 4,9-diamantanediol obtained in the step (ii) of Example 1, 0.10 g (0.5% relative to the mass of 4,9-diamantanediol) of phenothiazine (polymerization inhibitor), 200 g (2.78 moles, 10 times the mass of 4,9-diamantanediol) of acrylic acid (organic solvent), and 11.9 g (0.0908 mole, equal to the mole of 4,9-diamantandiol) of acrylic acid anhydride (polymerizable unsaturated carboxylic acid anhydride). The mixture was heated to 75°C.

Thereto was added 0.2 g (1% relative to the mass of 4,9-diamantanediol) of concentrated sulfuric acid (acid catalyst). The mixture was changed from a colorless state to a slightly reddish state and was in a suspension state. The mixture was stirred at 75°C for 6 hours. Then, GC analysis was conducted. The amount of remaining diamantanediol (raw material) was 8%, the amount of intended 9-hydroxy-4-diamantyl acrylate formed was 76%, and the amount of 4,9-di-ester compound (by-product) was 16%. The reaction mixture was a uniform solution.

The reaction mixture was cooled to room temperature. Thereto was added 200 g of methylene chloride (extraction solvent), followed by cooling to 5°C. Thereto was dropwise added 574.1 g of a 20 mass % aqueous sodium hydroxide solution while the temperature of the reaction mixture was maintained so as not to exceed 25°C. The organic layer and the aqueous layer were separated, and the organic layer was washed with 200 g of ion exchange water 4 times. The pHs of the organic layer and the aqueous layer after the washing were neutral.

To the solution was added 2 g (0.1 time the mass of 4,9-diamantanediol) of active carbon, followed by stirring at room temperature for 1 hour. Then, the active carbon was removed by filtration to obtain a nearly transparent solution of 9-hydroxy-4-diamantyl acrylate. To the solution was added 0.04 g (0.2% relative to the mass of 4,9-diamantanediol) of hydroquinone monomethyl ether, followed by distillation under reduced pressure, to obtain crude 9-hydroxy-4-diamantyl acrylate.

To the crude 9-hydroxy-4-diamantyl acrylate obtained was added 120g (6 times the mass of 4,9-diamantanediol) of toluene (crystallization solvent). The mixture was heated to 53°C for dissolution. The solution was cooled slowly to 2°c to separate out crystals. Aging was conducted at 2°C for 1 hour. The crystals were collected by filtration and dried to obtain 11 g (yield: 44%) of a white solid. The solid was analyzed by GC and GPC. As a result, the content of 9-hydroxy-4-diamantyl acrylate was 99% in terms of GC purity and the polystyrene-represented content of oligomer impurities was 0.2%.

### Example 11

Operations of steps (i) to (iii) were conducted in the same manner as in Example 1 to obtain crude 9-hydroxy-4-diamantyl methacrylate. Thereto was added 360 g (9 times the mass of 4,9-diamantanediol) of di-isopropyl ether (crystallization solvent). The mixture was heated to 55°C for dissolution. The resulting solution was cooled slowly to 2°C to separate out crystals. Aging was conducted at 2°C for 1 hour. The crystals were collected by filtration and dried to obtain 28 g (yield: 53%) of a white solid. The solid was analyzed by GC. As a result, the content of 9-hydroxy-4-diamantyl methacrylate was 77% in terms of GC purity.

### Example 12

Operations of steps (i) to (iii) were conducted in the same manner as in Example 1 to obtain crude 9-hydroxy-4-diamantyl methacrylate. Thereto was added 120 g (3 times the mass of 4,9-diamantanediol) of isobutyl acetate (crystallization solvent). The mixture was heated to 50°C for dissolution. The resulting solution was cooled slowly to 2°C to separate out crystals. Aging was conducted at 2°C for 1 hour. The crystals were collected by filtration and dried to obtain 22 g (yield: 42%) of a white solid. The solid was analyzed by GC. As a result, the content of 9-hydroxy-4-diamantyl methacrylate was 75% in terms of GC purity.

As seen from the comparison of Examples 11 and 12 with Example 1, use of an aromatic hydrocarbon as a crystallization solvent makes it possible to obtain a polymerizable hydroxydiamantyl ester compound of higher purity.

### Comparative Example 1

The inside of a 1000-ml, four-necked flask was purged with nitrogen sufficiently, after which nitrogen gas was blown into continuously. Into the four-necked flask were added 1 g (4.54 mmole) of the 4,9-diamantanediol obtained in the step (ii) of Example 1, 0.005 g (0.5% relative to the mass of 4,9-diamantanediol) of phenothiazine (polymerization inhibitor), 10 g (10 times the mass of 4,9-diamantanediol) of toluene (organic solvent), and 0.55 g (5.45 mmole, 1.2 times the mole of 4,9-diamantanediol) of triethylamine. The mixture was stirred at 40°C . Thereto was added 0.57 g (5.45 mmole, 1.2 times the mole of 4,9-diamantanediol) of methacrylic acid chloride. The reaction mixture stayed in a suspension state. Stirring was conducted at 40°C for 7 hours, after which GC analysis was conducted. As a result, the amount of remaining diamantanediol (raw material) was 85%, the amount of intended 9-hydroxy-4-diamantyl methacrylate formed was 1%, and the amount of by-product (unknown structure) was 4%. Thus, there was substantially no formation of intended 9-hydroxy-4-diamantyl methacrylate.

### Comparative Example 2

An operation was conducted in the same manner as in Example 7 except that, in the step (iii), no phenothiazine (polymerization inhibitor) was added. After stirring at 90°C for 7 hours, a large amount of insolubles (which were considered to be oligomer impurities and polymer impurities) appeared, and no isolation of intended 9-hydroxy-4-diamantyl methacrylate could be made.

### Comparative Example 3

An operation was conducted in the same manner as in Example 1 except that, in the step (iii), no methacrylic acid was used and 200 g of toluene (reaction solvent) was used. After stirring at 90°C for 4 hours, GC analysis was made. As a result, the amount of remaining diamantanediol was 41%, the amount of intended 9-hydroxy-4-diamantyl methacrylate formed was 17%, and the amount of 4,9-di-ester compound (by-product) was 42%. The reaction mixture was uniform.

Thus, when a reaction was made in an organic solvent without using any polymerizable unsaturated carboxylic acid having a solvent action as well, a large amount of a 4,9-di-ester compound was formed, and there was no selective formation of intended 9-hydroxy-4-diamantyl methacrylate.

## Claims

1. A method for producing a polymerizable hydroxydiamantyl ester compound represented by the following formula (1) 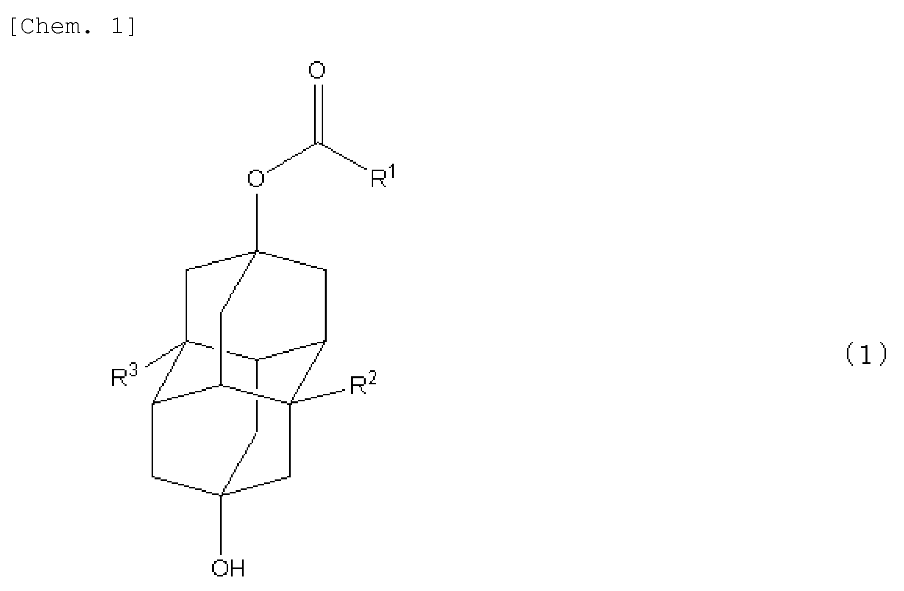 (wherein R¹ is a polymerizable unsaturated hydrocarbon group which may have a substituent, and R² and R³ are each independently a hydrogen atom or an alkyl group of 1 to 5 carbon atoms), which comprises:
(i) a step of di-halogenating a diamantane compound represented by the following formula (2) 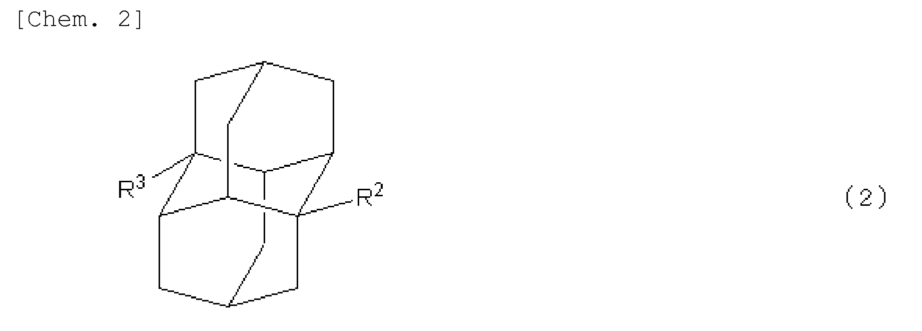 {wherein R² and R³ have each the same definition as for the R² and R³ in the above formula (1)} to obtain a 4,9-dihalogenated diamantane compound,
(ii) a step of hydrolyzing the 4,9-di-halgenated diamantane compound obtained in the above step, to obtain a 4,9-diamantanediol compound, and
(iii) a step of esterifying the 4,9-diamantanediol compound obtained in the above step, in a mixture of a polymerizable unsaturated carboxylic acid represented by the following formula (3) and a polymerizable unsaturated carboxylic acid anhydride represented by the following formula (4) 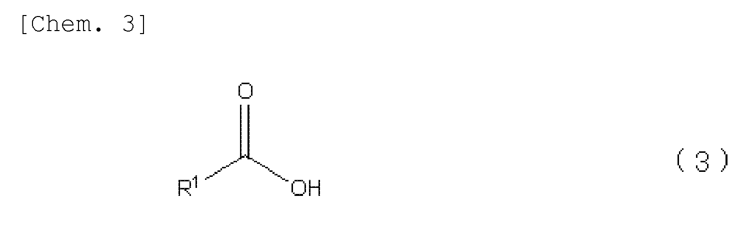 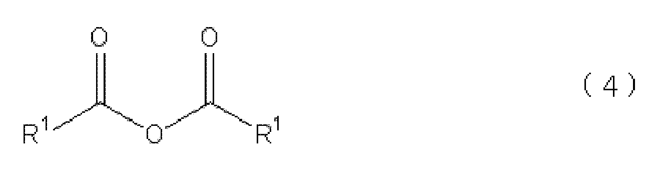 (wherein R¹_{S} are each a polymerizable unsaturated hydrocarbon group which may have a substituent, and they may be the same or different from each other) in the presence of a polymerization inhibitor and an acid catalyst, to obtain a crude product of polymerizable hydroxydiamantyl ester compound represented by the above formula (1).

2. The method for producing a polymerizable hydroxydiamantyl ester compound according to Claim 1, wherein, in the step (iii), the R¹ of the polymerizable unsaturated carboxylic acid and the two R¹S of the polymerizable unsaturated carboxylic acid anhydride are the same.

3. The method for producing a polymerizable hydroxydiamantyl ester compound according to Claim 1 or 2, wherein, in the steps (i) and the step (ii), the 4,9-dihalogenated diamantane compound is 4,9-dichlorodiamantane.

4. The method for producing a polymerizable hydroxydiamantyl ester compound according to any of Claim 1 to Claim 3, which further comprises:
a crystallization step of crystallizing the crude product of polymerizable hydroxydiamantyl ester compound obtained in the step (iii), using a solvent containing at least an aromatic hydrocarbon.
